# EUROPEAN PATENT APPLICATION

(11) **EP 4 289 946 A1**
(43) Date of publication of application: **13.12.2023**
(21) Application number: 22749117.2
(22) Date of filing: 28.01.2022
(51) Int. Cl.: C12N 9/10, C12N 15/54, C12N 15/70, C12N 1/21, C12P 13/00, C12R 1/19

(54) **CONSTRUCTION AND APPLICATIONS OF ENATIOSELECTIVE FLIPPED ?-TRANSAMINASE MUTANT**

(30) Priority: 05.02.2021 CN 202110162624; 24.05.2021 CN 202110567238
(71) Applicant: Zhejiang Apeloa Kangyu Pharmaceutical Co. Ltd., Zhejiang 322118 (CN); Apeloa Pharmaceutical Co., Ltd., Zhejiang 322118 (CN)
(72) Inventor: LIN, Shuangjun, Shanghai 200240 (CN); LIU, Qi, Shanghai 200240 (CN); DENG, Zixin, Shanghai 200240 (CN); HUANG, Tingting, Shanghai 200240 (CN)
(74) Representative: Perani & Partners S.p.A.
(86) International application number: PCT/CN2022/074737
(87) International publication number: WO 2022/166838

(57) **Abstract**

Provided is a transaminase mutant, having a sequence on which an amino acid mutation takes place on the basis of the sequence as represented by SEQ ID NO:13. Provided is an enantioselective flipped ω-transaminase ATA-117 mutant. The amino acid sequence of the mutant is as represented by SEQ ID NO:1; the nucleic acid sequence coding the mutant is as represented by SEQ ID NO:2. Also provided is a plasmid carrying the nucleic acid sequence, genetically engineered bacteria expressing the ω-transaminase ATA-117 mutant, and a method for producing (IR,2R)-1,3-dihydroxy-2-amino-1-p-methyl sulfonylphenylpropane with the ω-transaminase ATA-117 mutant

## Description

The present invention claims the priority of a Chinese patent application submitted on Feb. 5, 2021 (Application No.: 202110162624.3; Invention name: Construction and Applications of Enantioselective Flipped ω-Transaminase Mutant), and on May. 24, 2021 (Application No.: 202110567238.2; Invention name: Method for Synthesizing (1R, 2R)-AMPP By Using Enzyme Cascade Reaction), the contents of which are incorporated into the present invention by reference.

### Field of the Invention

The present invention relates to the enzyme engineering technology and the construction and applications of transaminase mutants, particularlyto an enantioselective flipped ω-transaminase mutant.

### Background of the Invention

Transaminases fall into two categories, i.e. α-transaminases and ω-transaminases. α-transaminases comprise type I, III and IV subgroups, which depend on the substrate ketone α-carboxylic acid for catalyzing transamination reaction. ω-transaminases belong to the type II subgroup and are independent of substrate ketone α-carboxylic acid and widely used in the asymmetric transamination of various aliphatic ketones and aromatic ketones to produce stereospecific amines. Among them, ATA117 is one of the widely used ω- transaminases. In 2008, Wolfgang Kroutil's research team reported that ATA117 could efficiently catalyze a series of aromatic ketones (e.g., 4-phenyl-2-butanone) and aliphatic ketones (e.g., octanone) to produce R-configuration amine with ee ≥ 99%. In 2010, Wolfgang Kroutil's research team reported that ATA117 could be used to catalyze organic selenium acetylbenzene to generate stereospecific R-p-seleno phenylethyl amine that is the chiral ligand of palladium catalyst. Through several iterations of the ATA117 enzyme, Gregory J. Hughes' research team was able to create the ATA-117-Rd11 mutant in the same year enabling them to catalyze the substrate prositagliptin ketone successfully, which has a strong steric hindrance. In 2020, Yu-Guo Zheng's research team used ATA-117-Rd11 mutant to catalyze the generation of stereospecific L-glufosinate ammonium products with ee>99% and 80 kg of the reaction precursors were transformed into 70 kg of L-glufosinate ammonium products within 24h. In conclusion, the ω-transaminase ATA117 has a broad spectrum of substrates and can be used to catalyze a variety of substrates to produce stereospecific amines efficiently.

In addition to producing stereospecific amines, ω-transaminases are provided with certain enantioselectivity to ortho chiral centers. In 2009, Wolfgang Kroutil's research team reported that R-4-phenyl-2-pyrrolidone was obtained through racemic aldehyde substrate kinetic resolution by ω-transaminase ATA117. ATA117 has an enantioselectivity to ortho-chiral centers of aldehydes. By optimizing the cosolvent and pH value, the *ee* value of its transamination product can reach up to 68%. In 2013, Vicente Gotor's research team examined the kinetic resolution of racemic α-alkyl-β-ketone esters of 24 commercial *S-* and *R-* transaminases. The result showed that a high catalytic efficiency could be achieved, but the diastereoselectivity was poor. In 2014, Wolfgang Kroutil's research team examined the kinetic resolution of the racemic 2-phenyl propionaldehyde of ω-transaminases from pseudomonas, arthrobacter, aspergillus terreus hyphomonas and the 2-phenyl propionaldehyde substituted by para meta ortho methyl and methoxy groups, characterizing the stereoselectivity of these transaminases to ortho chiral centers (*ee* (R)=76-98%). In 2018, Wolfgang Kroutil's research team examined the kinetic resolution of racemic aliphatic aldehyde of ω-transaminases from arthrobacter KNK168, aspergillus, fusarium graminearum, hyphomonas, Neosaetobacter fischeri and Bacillus megaterium, etc. The highly stereoselective antiepileptic drugs, i.e., Brivaracetam and Pregabalin, were obtained through a one-step transaminase reaction. Among them, for the ω-transaminases from hyphomonas, through the one-step kinetic resolution, a highly stereoselective chiral intermediate of *R*-Brivaracetam can be obtained, and the *ee* value can reach up to 92%. Wolfgang Kroutil's research team mutated several amino acid sites based on the fusarium graminearum sourced ω- transaminases and ArRmut11 mutant, respectively, and synthesized the S - pregabalin by catalyzing the racemic aliphatic aldehyde with the obtained mutant. The ee values can reach up to 76% and 80%, respectively. In 2019, Iván Lavandera's research team reported the kinetic resolution of racemic α-alkyl-β- ketoamide substrate for a series of commercial transaminases and Bacillus megaterium sourced transaminases and their mutant. Among which, the commercial *R*-transaminase has the highest catalytic efficiency and stereoselectivity for such substrate, and its diastereoselective *de* value can reach 96%. However, the Bacillus megaterium-sourced transaminases and their mutant can only convert part of the substrate, and their diastereoselectivity was low. In conclusion, for most of the ω- transaminases sourced from different screened species and genus as reported in the literature, high stereo selectivities for the ortho chiral centers of aldehyde substrates can be obtained, while for screen commercial transaminases, the high stereoselectivity of ortho chiral centers of ketone substrates such as α-alkyl-β-ketoamide can be obtained. However, no literature has reported the enantioselectivity of ω- transaminases to chiral aromatic hydroxyketones.

To quickly synthesize (1R, 2R)-amino diol products from chiral aromatic hydroxyketone molecules, it is imperative for individuals knowledgeable in this sector to find a highly enantioselective ω-transaminase.

### Description of the Invention

At present, providing a highly enantioselective ω-transaminase for chiral aromatic hydroxyketone compounds to synthesis (1R, 2R)-amino diol products via kinetic resolution is a problem that should be solved rapidly for individuals with expertise in this sector.

The present invention provides the following technical solutions:
[1]. A transaminase mutant which has amino acid mutation sequence as represented by SEQ ID NO: 13. The amino acid mutation Site is one of the following combinatorial mutation Sites: V69F, V69G, V69A, V69L, V69I, V69P, V69M, V69W, V69S, V69Q, V69T, V69C, V69N, V69Y, V69D, V69E, V69K, V69R, V69H, V69A+F122G, V69A+F122A, V69A+F122L, V69A+F122I, V69A+F122V, V69A+F122P, V69A+F122M, V69A+F122W, V69A+F122S, V69A+F122Q, V69A+F122T, V69A+F122C, V69A+F122N, V69A+F122Y, V69A+F122D, V69A+F122E, V69A+F122K, V69A+F122R, V69A+F122H, V69A+F122C+I157F, V69A+F122C+I157G, V69A+F122C+I157A, V69A+F122C+I157L, V69A+F122C+I157I, V69A+F122C+I157V, V69A+F122C+I157P, V69A+F122C+I157M, V69A+F122C+I157W, V69A+F122C+I157S, V69A+F122C+I157Q, V69A+F122C+I157T, V69A+F122C+I157C, V69A+F122C+I157N, V69A+F122C+I157Y, V69A+F122C+I157D, V69A+F122C+I157E, V69A+F122C+I157K, V69A+F122C+I157R, V69A+F122C+I157H, V69A+F122C+I157H+F225G, V69A+F122C+I157H+F225A, V69A+F122C+I157H+F225L, V69A+F122C+I157H+F225I, V69A+F122C+I157H+F225V, V69A+F122C+I157H+F225P, V69A+F122C+I157H+F225M, V69A+F122C+I157H+F225W, V69A+F122C+I157H+F225S, V69A+F122C+I157H+F225Q, V69A+F122C+I157H+F225T, V69A+F122C+I157H+F225C, V69A+F122C+I157H+F225N, V69A+F122C+I157H+F225Y, V69A+F122C+I157H+F225D, V69A+F122C+I157H+F225E, V69A+F122C+I157H+F225K, V69A+F122C+I157H+F225R, V69A+F122C+I157H+F225H.
[2]. The transaminase mutant described according to [1] is an enantioselective flipped ω-transaminase ATA117 mutant. The protein amino acid sequence of the said ω-transaminase ATA117 mutant is represented by SEQ ID NO: 1.
[3]. The transaminase mutant described according to [2], where the ω-transaminase ATA117 mutant is generated by the amino acid sequence of the ω-transaminase ATA117 through iterative saturation mutation. The ω-transaminase ATA117 mutant has at least a mutation of the valine, phenylalanine, isoleucine, or phenylalanine at Sites N1, N2, N3, or N4, respectively.
[4]. The transaminase mutant described according to [3], where the valine at Site N1 is mutated to alanine, the phenylalanine at Site N2 is mutated to cysteine, the isoleucine at Site N3 is mutated to histidine, and the phenylalanine at Site N4 is mutated to histidine.
[5]. A kind of DNA nucleotide sequence, where the said DNA nucleotide sequence contains a nucleotide sequence for coding a transaminase mutant as described in any one within [1]~[4].
[6]. A kind of DNA nucleotide sequence described according to [5], where the said DNA nucleotide sequence is that represented by SEQ ID NO: 2.
[7]. A kind of plasmid with the DNA nucleotide sequence as described according to[6].
[8]. A genetically engineered bacterium expressing the ω-transaminase ATA117 mutant, where the protein amino acid sequence of the ω-transaminase ATA117 mutant is represented by SEQ ID NO: 1.
[9]. A method for constructing a genetically engineered bacterium expressing the ω-transaminase ATA117 mutant is described according to [8]. It includes the following steps: A1, Taking the carried recombinant plasmid for coding the gene of ATA117 as the template; A2, designing and synthesizing the primer containing the mutation; A3, Performing a reverse PCR amplification of the whole-plasmid; and A4, Transforming the PCR product to express the host.
[10]. A method for constructing a genetically engineered bacterium expressing the ω-transaminase ATA117 mutant described according to [9], where the expression host is *E.coli* BL21 (DE3).
[11] A method for producing (1R, 2R) -1,3-dihydroxy-2-amido-1-p-methyl sulfonyl phenyl propane based on the transaminase mutant described according to any item within [1]~[4]: With the pure protein of the transaminase mutant as the catalyst, the racemization of 1,3-dihydroxy-1-p-methyl sulfonyl propiophenone can be catalyzed in a buffer system. In addition, the high stereoselective (1R, 2R) -1,3-dihydroxy-2-amido-1-p-methyl sulfonyl phenylpropane can be obtained through kinetic resolution.

Further, to solve the above technical problems, the present invention is based on the molecular modification of ω-transaminase ATA117 by semi-rational design, combined with the iterative saturation mutation method of the gene to obtain the corresponding mutants.

The present invention, firstly, provides a transaminase mutant, which has amino acid mutation sequence as represented by SEQ ID NO: 13, and the said amino acid mutation is one of the following mutations: V69F, V69G, V69A, V69L, V69I, V69P, V69M, V69W, V69S, V69Q, V69T, V69C, V69N, V69Y, V69D, V69E, V69K, V69R, V69H, V69A+F122G, V69A+F122A, V69A+F122L, V69A+F122I, V69A+F122V, V69A+F122P, V69A+F122M, V69A+F122W, V69A+F122S, V69A+F122Q, V69A+F122T, V69A+F122C, V69A+F122N, V69A+F122Y, V69A+F122D, V69A+F122E, V69A+F122K, V69A+F122R, V69A+F122H, V69A+F122C+I157F, V69A+F122C+I157G, V69A+F122C+I157A, V69A+F122C+I157L, V69A+F122C+I157I, V69A+F122C+I157V, V69A+F122C+I157P, V69A+F122C+I157M, V69A+F122C+I157W, V69A+F122C+I157S, V69A+F122C+I157Q, V69A+F122C+I157T, V69A+F122C+I157C, V69A+F122C+I157N, V69A+F122C+I157Y, V69A+F122C+I157D, V69A+F122C+I157E, V69A+F122C+I157K, V69A+F122C+I157R, V69A+F122C+I157H, V69A+F122C+I157H+F225G, V69A+F122C+I157H+F225A, V69A+F122C+I157H+F225L, V69A+F122C+I157H+F225I, V69A+F122C+I157H+F225V, V69A+F122C+I157H+F225P, V69A+F122C+I157H+F225M, V69A+F122C+I157H+F225W, V69A+F122C+I157H+F225S, V69A+F122C+I157H+F225Q, V69A+F122C+I157H+F225T, V69A+F122C+I157H+F225C, V69A+F122C+I157H+F225N, V69A+F122C+I157H+F225Y, V69A+F122C+I157H+F225D, V69A+F122C+I157H+F225E, V69A +F 1 22C+I 157H+F225K, V69A+F122C+I157H+F225R, V69A+F122C+I157H+F225H.

The present invention, secondly, provides an enantioselective flipped ω-transaminase ATA117 mutant, whose protein amino acid sequence is represented by SEQ ID NO: 1 (amino acid mutation V69A+F122C+I157H+F225H occurs based on the sequence represented by SEQ ID NO: 13).

The sequences represented by SEQ ID NO: 1 are as follows:

MAFSADTSEI VYTHDTGLDY ITYSDYELDP ANPLAGGAAW IEGAFVPPSE ARISIFDQGY 60
LHSDVTYTAF HVWNGNAFRL DDHIERLFSN AESMRIIPPL TQDEVKEIAL ELVAKTELRE 120
ACVSVSITRG YSSTPGERDI TKHRPQVYMY AVPYQWHVPF DRIRDGVHAM VAQSVRRTPR 180
SSIDPQVKNF QWGDLIRAVQ ETHDRGFEAP LLLDGDGLLA EGSGHNVVVI KDGVVRSPGR 240
AALPGITRKT VLEIAESLGH EAILADITLA ELLDADEVLG CTTAGGVWPF VSVDGNPISD 300
GVPGPVTQSI IRRYWELNVE SSSLLTPVQY 330

Further, this mutant is generated by the amino acid sequence of ATA117 through iterative saturation mutation. It has at least a valine, phenylalanine, isoleucine or phenylalanine mutation at Sites N1, N2, N3 or N4, respectively.

Further, the valine at Site N1 is mutated to alanine

Further, the phenylalanine at Site N2 is mutated to cysteine.

Further, the isoleucine at Site N3 is mutated to histidine.

Further, the phenylalanine at Site N4 is mutated to histidine.

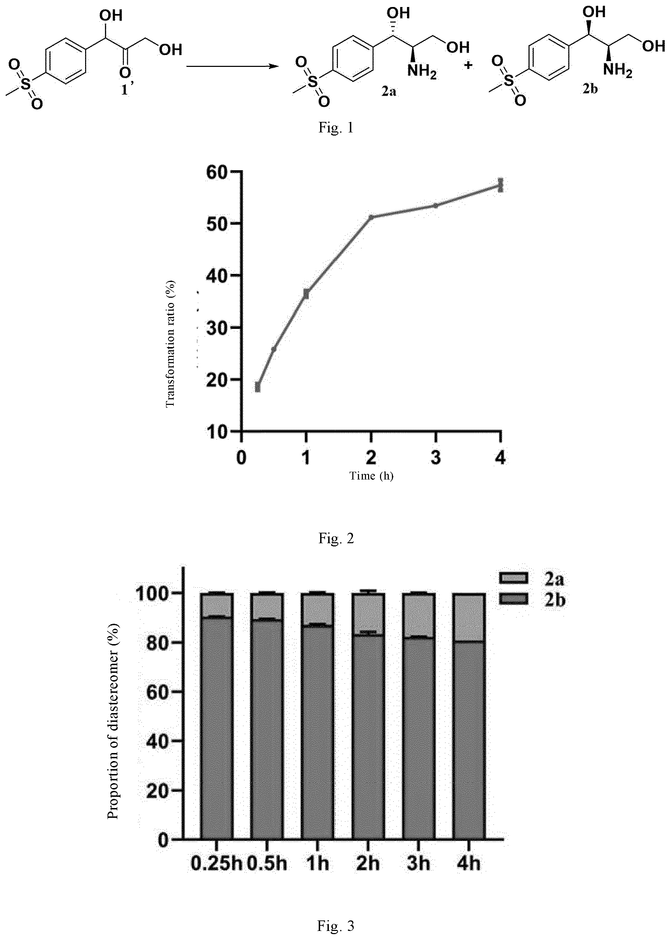

Further, this mutant's racemic p-methyl sulfonyl phenyl dihydroxyketone (Formula 1' in Fig. 1) has a high R-enantioselectivity.

Third, The present invention provides a kind of DNA containing a nucleotide sequence for coding this ω-transaminase ATA117 mutant.

Further, the DNA nucleotide sequence is represented by SEQ ID NO: 2.

The sequences represented by SEQ ID NO: 2 are as follows:

atggcattta gcgcggatac cagcgaaatc gtctataccc acgataccgg tctggattac 60
atcacctaca gcgattacga actggatccg gcaaatccgt tagcaggcgg cgcagcttgg 120
attgaaggcg catttgttcc gccgtctgaa gcgcgtatta gcatcttcga tcagggctat 180
ctgcatagcg acgttaccta taccgcattc cacgtttgga acggtaacgc atttcgtctg 240
gacgatcaca tcgaacgtct gttcagcaac gccgaaagta tgcgtattat tccgccgctg 300
acccaagacg aagtcaaaga aatcgcgctg gaactggttg caaaaaccga actgcgcgaa 360
gcgtgtgtta gcgttagcat tacccgcggt tatagtagta ccccgggcga acgcgatatt 420
accaaacatc gtccgcaggt ctacatgtac gctgttccgt accagtggca tgttccgttt 480
gatcgtattc gcgacggcgt tcacgcaatg gttgcacaga gcgtgcgtcg taccccgcgt 540
agcagcatcg atccgcaggt caaaaacttc cagtggggcg atttaattcg cgcagttcag 600
gaaacccacg atcgcggttt tgaagcaccg ttactgttag acggcgacgg tttactggca 60
gaaggtagcg gccataacgt cgtcgtcatt aaagatgggg ttgttcgttc tccgggtcgt 720
gcagcattac cgggtattac ccgcaaaacc gttctggaaa ttgcggaatc cctgggtcat 780
gaagcgattc tggcggatat taccttagcg gaactgctgg acgcagacga agttttaggt 840
tgtaccaccg ctggcggcgt ttggccgttt gttagcgttg acggcaatcc gattagcgat 900

ggtgttccgg gtccggttac ccaaagcatt attcgtcgct actgggagct gaacgttgaa 960
agtagcagcc tgttaacccc ggttcagtat taa 993

The present invention, fourthly, provides a kind of plasmids carrying the nucleotide mentioned above sequences.

Further, SYNBIO Technologies obtain the recombinant plasmids carrying the gene for coding ω-transaminase ATA117 after ATA117 gene synthesis, restriction enzyme digestion, ligation reaction, and pRSFduet-1 vector insertion at the salI/HindIII Digestion Site.

The present invention, fifthly, provides a genetically engineered bacterium expressing the ω-transaminase ATA117 mutant.

Further, the method for constructing the genetically engineered bacterium includes the following steps: A1, Taking the carried recombinant plasmid for coding the gene of ATA117 as the template; A2, designing and synthesizing the primer containing the mutation; A3, Performing a reverse PCR amplification of the whole-plasmid; and A4, Transforming the PCR product to express the host.

Further, the expression host is *E.coli* BL21 (DE3).

The sixth aspect of the present invention provides a method for producing (1R, 2R) -1,3-dihydroxy-2-amido-1-p- methyl sulfonyl phenyl propane (Formula 2b shown in Fig. 1) through the ω-transaminase ATA117 mutant: With pure protein of the transaminase mutant as the catalyst, the racemization of 1, 3-dihydroxy- 1-p-methyl sulfonyl propiophenone (Formula 1' shown in Fig. 1) can be catalyzed in a buffer system. And the high stereoselective (1R, 2R) -1, 3-dihydroxy-2-amido-1-p-methyl sulfonyl phenylpropane can be obtained through kinetic resolution (Formula 2b shown in Fig. 1.)

Further, the buffer system is a single aqueous phase system.

In the present invention, (1R, 2R) -1, 3-dihydroxy-2-amido-1-p-methyl sulfonyl phenylpropane can also be called as (1R, 2R) -2-amido-1-(4- (methylsufonyl) phenyl) propane 1, 3-diol, i.e. (1R, 2R) -AMPP.

Invention effect:
1. The ATA117_ACHH mutant obtained in the present invention has a high R-selectivity for ortho chiral centers of aromatic hydroxyketones, which helps to complement the study on the enantioselectivity of chiral hydroxy ketones from ω-transaminase.
2. The kinetic resolution of the ATA117_ACHH mutant to racemic hydroxyketone compounds can help to obtain highly stereoselective (1R, 2R)-amino alcohols, which are chiral intermediates of various drugs such as thiamphenicol, florfenicol, and pseudoephedrine. Therefore, this mutant is quite suitable for the synthesis of intermediates of chiral drugs.
3. The enantiomeric ratio of the wild ATA117 to the chiral 1,3-dihydroxy-1-p-methyl sulfonyl phenylacetone 1 is 8.5 E (S), while the enantiomer ratio of the ATA117_ACHH mutant is 11.7 E (R). The obtained mutant lays a foundation for its application in synthesizing chiral (1R, 2R) - amino diol products.

### Brief Description of the Figures

Fig. 1 shows the kinetic resolution schematic of the ATA117 _ACHH mutant to compounds (Formula 1 ');
Fig. 2 shows the transformation ratio schematic for racemic substrate (Formula 1') synthesis Formula 2a and 2b after the kinetic resolution of ATA117 _ACHH mutant protein
Fig. 3 shows the stereoselectivity schematic for racemic substrate (Formula 1') synthesis Formula 2a and 2b after the kinetic resolution of ATA117_ACHH mutant protein
Fig. 4 shows the liquid phase of the reaction products obtained by taking p-methyl sulfonyl benzaldehyde as the substrate under C18 column conditions;
Fig. 5 shows the liquid phase of the threo reaction products obtained by taking p-methyl sulfonyl benzaldehyde as the substrate under chiral liquid phase conditions.
Fig. 6 shows the H-NMR of the reaction product (1R, 2R)-AMPP.
Fig. 7 shows the C-NMR of the reaction product (1R, 2R)-AMPP;
Fig. 8 shows the liquid phase of the reaction products obtained by taking benzaldehyde as the substrate under C18 column conditions, i.e., the high-performance liquid chromatography identification of the benzaldehyde reaction products obtained through enzyme cascade catalysis. Among which, A is the HPLC chromatography of the enzyme cascade reaction products, B is the standard (1R, 2R)-phenylserinol and C is the standard mixture of threo and erythro products.
Fig. 9 shows the liquid phase of the reaction products obtained by taking p-methyl benzaldehyde as the substrate under C18 column conditions, i.e., the high-performance liquid chromatography identification of the p-methyl benzaldehyde reaction products obtained through enzyme cascade catalysis. Among which A is the HPLC chromatography of the enzyme cascade reaction products, B is the standard (1R, 2R)-p-methyl phenylserinol and C is the standard mixture of threo and erythro products.

### Detailed Description of the Preferred Embodiments

The present invention is further described with reference to the following Embodiments and data. It should be understood that these Embodiments are only intended as examples to illustrate the present invention and not to limit the scope of the invention in any way.

General description of the source of the biological materials described in the present invention:
The (1R, 2R) -1, 3-dihydroxy-2-amino-1-methyl sulfonyl phenylpropane products were purchased from Huangshi Yongxin Biotechnology Co., Ltd. The FLM Inc. supplied the standard liquid chromatography column C18 (4.6 × 250mm, particle size 5µm). Daicel Chiral Technologies (China) CO., LTD. supplied the chiral liquid chromatography column IG (4.6 × 250mm, particle size 5µm).

In this Specification, the methyl sulfonyl, methyl sulphonyl, and methyl groups represent the same chemical structure.

### Embodiment 1 Iterative saturation mutation

| | |
|---|---|
| VN-F: | agcgacgttacctatacc**nnk**ttccacgtttggaacggtaacgca (SEQ ID NO: 3) |
| VN-R: | accgttccaaacgtggaa**mnn**ggtataggtaacgtcgctatgcag (SEQ ID NO: 4) |
| FN-F: | accgaactgcgcgaagcg**nnk**gttagcgttagcattacccgcggt (SEQ ID NO: 5) |
| FN-R: | ggtaatgctaacgctaac**mnn**cgcttcgcgcagttcggtttttgc (SEQ ID NO: 6) |
| IN-F: | gctgttccgtaccagtgg**nnk**gttccgtttgatcgtattcgcgac (SEQ ID NO: 7) |
| IN-R: | aatacgatcaaacggaac**mnn**ccactggtacggaacagcgtacat (SEQ ID NO: 8) |
| FN-F: | ctggcagaaggtagcggc**nnk**aacgtcgtcgtcattaaagatggg (SEQ ID NO: 9) |
| FN-R: | tttaatgacgacgacgtt**mnn**gccgctaccttctgccagtaaacc (SEQ ID NO: 10) |

N refers to any base within a, t, c, and g, and k refers to base t or g. This degenerate codon can be used for coding 20 amino acids at random.
(1) With the recombinant plasmid pRSFduet-1-ATA117 as the template, VN-F and VN-R as the primers, use the Phanta Max polymerase (purchased from Vazyme Biotech) for PCR amplification (95°C for 5min; 95°C for 30s; 65°C for 30 s; 72°C for 7.5min; 34 cycles; 72°C for 10min). Transform the PCR product directly into E. *coli* BL21 (DE3) receptive cells after FD-Dpn I digestion (incubator at 30°C, 6h). After the resuscitation fluid has been blown and suctioned for thorough and equal mixing, apply about 1/8 of the resuscitation fluid on a kanamycin-resistant LB plate. Culture it at 37°C for 12-16h to obtain a recombinant sub library.
(2) Select all the single colonies (about 40) from the dilution mentioned above the plate and culture them in kanamycin-resistant LB medium at 37°C for 7-8h. Then, use part of the medium for sequencing and temporarily put the other part in a 4°C refrigerator for short-term storage. Transfer the correctly sequenced transformant medium to a 24-hole deep hole plate containing fresh 5mL LB medium with kanamycin resistance at 1% inoculation amount. After 3h of cultivation at 37 °C, add 0.2mM IPTG inducer to induce efficient expression of the recombinant gene at 20°C for 15h. Centrifuge the recombinant cells at 4000rpm to collect thalli and store them at -80°C. Finally, use the same induction method to obtain recombinant thallus pRSFduet-1-ATA117 for control.
(3) Add the obtained recombinant thalli to the 300µl 50mM Tris-Cl (pH=7.5) for suspension and ultrasonic fragmentation, and centrifuge it at a low temperature to get the supernatant to be used for the catalytic racemization of 1,3-dihydroxy-1-p-methyl sulfonyl propiophenone.
(4) Measure the diastereoselectivity of the reaction product described in (3) through a C18 column. Embodiment 2 displays the conditions for the liquid phase analysis. The experimental results indicate that among the ATA117/VN saturated mutants, the selectivity of the ATA117_A to S-hydroxyketone substrate is significantly reduced compared with that of the wild-type ATA117, so ATA117_A is chosen as the template for the subsequent FN saturation mutation. The mutant construction, expression, response, and detection methods are described above. The experimental results indicate that in the ATA117A/FN saturated mutants, the selectivity of the ATA117_AC mutant is flipped to the chiral hydroxyketone substrate, so ATA117_ AC is chosen as the template for the subsequent IN saturation mutation. After the IN saturation mutation, the iterative FN mutation can be performed to obtain the ATA117_ACHH mutant with high selectivity to the R-hydroxyketone substrate.

### Embodiment 2 Expression and purification of transaminase

Expression and purification method of transaminase: Select single colonies of *E. coli* BL21 (DE3) containing recombinant plasmids from the LB solid medium and inoculate it to a 40 ml LB liquid medium (with 50µg/ml kanamycin antibiotic), perform an overnight culture at 37°C and 220rpm. Transfer 7.5ml of bacterial culture medium to 2L shake flasks containing 500 ml of LB liquid medium, inoculate it in two bottles, go on to perform a culture at 37°C until the OD600 reaches 0.6-0.8, add 0.2mM IPTG for induction, and induce the culture at 20 °C and 200 rpm for 15h. Collect 1L of fermentation broth and centrifuge it at 5000 rpm for 20min to collect cells. Resuspend the collected cells in a 30mL nickel column-binding buffer and place them in an ice-water mixture for ultrasonic fragmentation. Ultrasonic fragmentation conditions: operating for 5s and then pausing for 10s. Total time: 30 min. Centrifuge the mixture after fragmentation at 12000 rpm for 1h, and filter the supernatant through 0.22µm membrane filtration. Then load the filtered sample with 2ml of nickel filler pre-balanced with nickel column binding buffer. Wash the heteroproteins with 50 mM imidazole elution buffer (10 times the column volume), and then elute the target protein with 5 ml of 250 mM imidazole elution buffer. Collect samples with different tubes (500µl/tube). Determine the protein concentration of each tube with a NanoDrop meter, combine several tubes of protein solution with higher concentration, dilute or concentrate it to 2.5 ml, load the desalting column sample balanced by glycerol buffer, after the protein solution is drained and add 3.5ml glycerol buffer to elute the protein.

### Embodiment 3 Enantioselectivity determination of ATA117 and its mutants

The volume of the reaction system is 100µl (50mM Tris-Cl buffer, pH 7.5) in total, including 2mM racemic 1,3-dihydroxy-1-p-methyl sulfonyl propiophenone, 2mM PLP, 200mM D-Ala, 5µM ATA117, 20µM ATA117_A, 20µM ATA117_AC, 40µM ATA117_ACH, 40µM ATA117_ACHH. Perform a reaction at 30°C for 1h, and add 200µl methanol to terminate the reaction. The sample is analyzed through the Agilent liquid phase, C18 column (4.6 × 150 mm, particle size 3µm). Column temperature 30°C, 224nm, and 0.5ml/min, Phase A: H₂O (10mM KH₂PO₄, pH = 8.5), Phase B: acetonitrile. The chromatographic conditions are shown in Table 1:

**Table 1 Chromatographic conditions**

| Time | Proportion of Phase B |
|---|---|
| 0min | 2% |
| 15min | 10% |
| 18min | 30% |
| 24min | 30% |
| 25min | 2% |
| 31min | 2% |

The retention time results for the amination products of (1S, 2R) - and (1R, 2R) -1, 3-dihydroxy-1-p-methylsulfonyl propiophenone are 13.2 min and 14.1 min, respectively. *de* = (RR SR)/(RR+SR), *E*=ln [1-c × (1+*de*)]/ln [1-c × (*1-de*)]*.* Where: RR and SR refer to the amination product concentration of (1R, 2R) - and (1S, 2R) -1, 3-dihydroxy-1-p-methylsulfonyl, propiophenone, and c refers to the transformation ratio of the racemic 1, 3-dihydroxy-1-p-methyl sulfonyl propiophenone.

The test results are shown in Table 2:

**Table 2 Enantiomeric ratio of ATA117 and its mutant (E)**

| Substrate conc. (mM) | Enzyme | Enzyme conc. (µM) | Time (h) | Transformation ratio (%) | *De* (%) | *E* |
|---|---|---|---|---|---|---|
| 2 | ATA117 | 5 | 1 | 38 | 70 | 8.5(S) |
| 2 | ATA117_A (V69A) | 20 | 1 | 47 | 40 | 3.2(S) |
| 2 | ATA117_AC (V69A+F122C) | 20 | 1 | 29 | 15 | 1.4(R) |
| 2 | ATA117_ACH (V69A+F122C+I157H) ATA117_ACHH | 40 | 1 | 40 | 70 | 8.8(R) |
| 2 | (V69A+F122C+1157H+F225H) | 40 | 1 | 21 | 81 | 11.7(R) |

### De refers to the diastereomeric excess value

### Embodiment 4 Application of ATA117_ACHH in synthesizing (1R, 2R) -1,3-dihydroxy-2-amino-1-p-methyl sulfonyl phenylpropane

The volume of the reaction system is 100µl (100mM Tris-Cl buffer, pH 7.5) in total, including 25µM ATA117_ACHH pure protein, 5mM racemic 1,3-dihydroxy-1-p-methyl sulfonyl propiophenone, 2mM PLP, 200mM D-Ala, 10mM NADH, 90U/ml LDH, 200mM glucose, 30U/ml GDH. Perform a reaction at 30°C for 15min, 30min, 1h, 2h, 3h, 4h, respectively, and then add methanol to terminate the reaction. A common liquid column should first separate the reaction product to examine the diastereoisomer selectivity. The analytical method is as described above. The retention time results for the amination products of (erythro) - and (threo) -1, 3-dihydroxy-1-p-methylsulfonyl propiophenone (i.e., 1, 3-dihydroxy- 2-amido-1-p-methyl sulfonyl phenylpropane) are 13.2min and 14.1min, respectively. According to the liquid phase peaks, collect the sample of (threo) -1, 3-dihydroxy-2-amino-1-p-methyl sulfonyl phenylpropane, concentrate it, and then separate it with a chiral liquid column to examine the enantiomer selectivity. The analytical conditions of enantiomers are: Chiral column IG, column temperature 25°C, 0.5ml/min, and 224nm. Chromatographic conditions: pure methanol (containing 0.1% Diethylamine), 15 min. The retention time results for (1R, 2R) - and (1S, 2S) -1, 3-dihydroxy-2-amino-1-methyl sulfonyl phenylpropane are 5.6 and 9.4 minutes, respectively. ee = [(RR SS)/ (RR+SS)] × 100%, where RR (Formula 2b) and SS refer to the (1R, 2R) - and (1S, 2S) -1, 3-dihydroxy-2-amino-1-p-methyl sulfonyl phenylpropane. The transformation ratio for ATA117_ACHH mutant protein in catalyzing the synthesis of (1R, 2R) -1, 3-dihydroxy-2-amino-1-p-methyl sulfonyl phenylpropane (Formula 2b) and the stereoselectivity are shown in Fig. 2 and 3.

### Embodiment 5 Application of ATA117_ACHH and mutants in synthesizing (1R, 2R)-AMPP and their derivatives.

For the synthesis method of (1R, 2R)-AMPP and its derivatives, with the benzaldehyde derivative as the substrate, through the transketo reaction under the action of the E. coli transketolase mutant, the reaction products shown in Formula 2 are obtained. Then with the compound shown in Formula 2 as the substrate, the transamination reaction is carried out under the action of the transaminase ATA117 mutant. The amino donors are D-alanine, D-glycine, D-valine, D-leucine, D-isoleucine D-methionine, D-proline, D-tryptophan, D-serine, D-tyrosine, D-cysteine, D-phenylalanine, D-asparagine, D-glutamine, D-threonine, D-aspartic acid, D-glutamic acid, D-lysine, D-arginine, D-histidine or isopropylamine, the (1R, 2R)-AMPP and its derivatives shown in Formula 3 are obtained through their synthesis.

The reaction formulas involved are as follows:

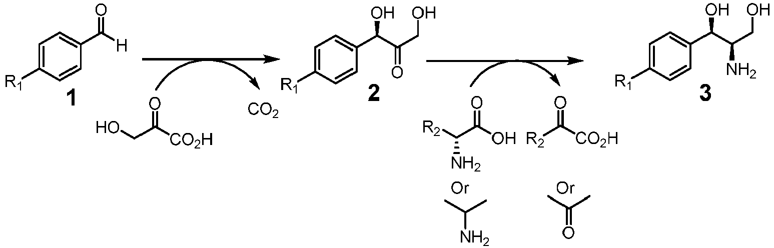

The following is a detailed explanation of this method:

### Step 1: Expression and purification of E. coli transketolase mutant

Introduce the nucleotide sequence with the above-mentioned amino acid mutation in the sequence represented by SEQ ID NO: 12 into Vector pET28a and host E.coli BL21. Then, select the single colony of E. coli BL21 containing recombinant plasmids from the LB solid medium and inoculate them to a 40 ml LB liquid medium (with 50µg/ml kanamycin antibiotic), and perform an overnight culture at 37°C and 220rpm. Finally, transfer 10 ml of bacterial culture medium to a 2L shake flask containing 500 ml of LB liquid medium, inoculate two bottles, go on to perform a culture at 37°C and 220 rpm until the OD600 reaches 0.6-0.8, add 0.2mM IPTG for induction, and induce the culture at 30 °C and 200 rpm for 5h.

After the cultivation, collect 1L of fermentation broth and centrifuge it at 5000 rpm for 20min to collect cells. Resuspend the collected cells in a 30 mL nickel column-binding buffer and place them in an ice-water mixture for ultrasonic fragmentation. Ultrasonic fragmentation conditions: Operating for 5s and then pausing for 10s. Total time: 30 min.

Centrifuge the mixture after fragmentation at 12000 rpm for 30min (e.g., 30min or 1h), and filter the supernatant through 0.22µm membrane filtration. Then, load the filtered sample with 2ml of nickel filler pre-balanced with nickel column binding buffer. Wash the heteroproteins with 50 mM imidazole elution buffer (20 times the column volume), and then elute the target protein with 5 ml of 250 mM imidazole elution buffer. Collect samples with different tubes (500µl/tube). Determine the protein concentration of each tube, combine several tubes of protein solution with higher concentration, dilute or concentrate it to 2.5 ml, and load the desalting column sample balanced by glycerol buffer. After the protein solution is drained, add 3.5ml glycerol buffer to elute the protein. Thus, the pure protein of E. coli transketolase mutant is obtained.

In Step 1, the protein concentration can be measured using a Thermo Scientific Nanodrop 8000 detector to detect the absorbance value E at 280nm. The target protein concentration can be converted according to the molar extinction coefficient, i.e., the protein concentration (mg/mL) = E/molar extinction coefficient. The software Vector NTI can predict the molar extinction coefficient of the recombinase.

### Step 2: Expression and purification of transaminase ATA117 mutant

Introduce the nucleotide sequence with the above-mentioned amino acid mutation in the sequence represented by SEQ ID NO: 14 into Vector pRSFDuet and host E.coli BL21. Then select the single colony of E. coli BL21 containing recombinant plasmids from the LB solid medium and inoculate them to a 40 ml LB liquid medium (with 50µg/ml kanamycin antibiotic), and perform an overnight culture at 37°C and 220rpm. Finally, transfer 10 ml of bacterial culture medium to a 2L shake flask containing 500 ml of LB liquid medium, inoculate two bottles, go on to perform a culture at 37°C and 220 rpm until the OD600 reaches 0.6-0.8, add 0.2 mM IPTG for induction, and induce the culture at 20°C and 200 rpm for 15h.

After the cultivation, collect 1L of fermentation broth and centrifuge it at 5000 rpm for 20min to collect cells. Resuspend the collected cells in a 30 mL nickel column-binding buffer and place them in an ice-water mixture for ultrasonic fragmentation. Ultrasonic fragmentation conditions: Operating for 5s and then pausing for 10s. Total time: 30 min.

Centrifuge the mixture after fragmentation at 12000 rpm for 30min, and filter the supernatant through 0.22µm membrane filtration. Then load the filtered sample with 2ml of nickel filler pre-balanced with nickel column binding buffer. Wash the heteroproteins with 50 mM imidazole elution buffer (10 times the column volume), and then elute the target protein with 5 ml of 250 mM imidazole elution buffer. Collect samples with different tubes (500µl/tube). Since the transaminase binds cofactor PLP, it is yellow. Combine several dark yellow protein solution tubes, dilute or concentrate the protein solution to 2.5 ml, and load the chromatographic column sample balanced by glycerol buffer. Then, add 3.5 ml of glycerol buffer after the drained protein solution to elute the protein, t. Thus, it is possible to extract transaminase protein in its purest form.

### Step 3: Synthesis of (1R, 2R)-AMPP and its derivatives

Add 10mM benzaldehyde derivative, 30mM lithium hydroxypyruvate (LiHPA, as a transketotic donor), 4.8mM thiamine pyrophosphate (TPP, as the cofactor for the transketo reaction), 18 mM MgCl₂ (as the metal ions for the transketotic reaction), and 100µM E. coli transketolase mutant in a 50µl Tris-HCl buffer containing 100mM (pH 7.5) for the reaction for 1-3h at 25°C.

For the reaction mentioned above system, add 100mM Tris-HCl buffer (pH 7.5), 200mM D-Ala, or D-glycine, D-valine, D-leucine, D-isoleucine, D-methionine, D-proline, D-tryptophan, D-serine, D-tyrosine, D-cysteine, D-phenylalanine, D-asparagine, D-glutamine, D-threonine, D-aspartic acid, D-glutamic acid, D-lysine, D-arginine, D-histidine or isopropylamine (as a transamination donor), 2 mM pyridoxal phosphate (PLP, as the cofactor for the transamination reaction), 50µM transaminase ATA117 mutant, and expand the reaction system to 100µl. The (1R, 2R) - AMPP and its derivatives are obtained after the reaction lasts for 3-6h at 25°C.

**Table 1 Transformation ratio and stereoselectivity of the cascade reaction products**

| Embodiment | Substrate | Amino donor | E. coli transketolase | Transaminase | Transformation ratio | *de* | *ee* |
|---|---|---|---|---|---|---|---|
| 1 | P-methyl sulfonyl benzaldehyde | D-alanine | H26Y | V69A+F122C+I157H | *** | * | ***** |
| 2 | P-methyl sulfonyl benzaldehyde | D-alanine | H26Y+F434Y | V69A+F122C+I157H | *** | *** | ***** |
| 3 | P-methyl sulfonyl benzaldehyde | D-alanine | H26Y+F434Y+L466H | V69A+F122C | *** | *** | ***** |
| 4 | P-methyl sulfonyl benzaldehyde | D-alanine | H26Y+F434Y+L466H | V69A+F122C+I157H | *** | **** | ***** |
| 5 | P-methyl sulfonyl benzaldehyde | D-alanine | H26Y+F434Y+L466H | V69A+F122C+I157H+F225H | *** | **** | ***** |
| 6 | P-methyl sulfonyl benzaldehyde | Isopropylamine | H26Y+F434Y | V69A+F122C+I157H | *** | *** | ***** |
| 7 | P-methyl sulfonyl benzaldehyde | Isopropylamine | H26Y+F434Y+L466H | V69A+F122C+I157H | *** | **** | ***** |
| 8 | Benzaldehyde | D-alanine | H26Y+F434Y | V69A+F122C+I157H | ** | *** | ***** |
| 9 | Benzaldehyde | D-alanine | H26Y+F434Y+L466F | V69A+F122C+I157H+F225H | ** | **** | ***** |
| 10 | Benzaldehyde | Isopropylamine | H26Y+F434Y | V69A+F122C+I157H | ** | *** | ***** |
| 11 | Benzaldehyde | Isopropylamine | H26Y+F434Y+L466F | V69A+F122C+I157H | ** | **** | ***** |
| 12 | P-methyl benzaldehyde | D-alanine | H26Y+F434Y | V69A+F122C+I157H | ** | *** | ***** |
| 13 | P-methyl benzaldehyde | D-alanine | H26Y+F434Y+L466F | V69A+F122C+I157H+F225H | ** | **** | ***** |
| 14 | P-methyl benzaldehyde | Isopropylamine | H26Y+F434Y | V69A+F122C+I157H | ** | *** | ***** |
| 15 | P-methyl benzaldehyde | Isopropylamine | H26Y+F434Y+L466F | V69A+F122C+I157H | ** | **** | ***** |

| | | | | | | | |
|---|---|---|---|---|---|---|---|
| *: Transformation ratio within 0-40%; **: Transformation ratio within 40-60%; ***: Transformation ratio within 60-80%; ****: Transformation ratio being >80%. *: *de* within 0-40%; **: *de* within 40-70%; ***: *de* within 70-90%; ****: *de* being >90%. *: *ee* within 0-40%; **: *ee* within 40-70%; ***: *ee* within 70-90%; **** *ee* within 90-99%; *****: *ee* being >99%. | | | | | | | |

Further, the present invention detects the reaction products through Agilent 1200 liquid chromatography. The specific testing methods include: Using a C18 column (4.6x150mM, particle size 3µm); Column temperature: 30 °C, 0.5 ml/min; Phase A: H₂O (10mM KH₂PO₄, pH = 8.5); Phase B: Acetonitrile.

**Table 4 Liquid chromatography conditions**

| Product | | | HPLC method | Wavelength (nm) | Retention time (min) |
|---|---|---|---|---|---|
| P-methyl sulfonyl phenylserinol | | | | 224 | |
| | Time | | Phase B gradient | | *Erythro*: 13.4 |
| | | 0min | 2% | | *Threo*: 14.2 |
| | | 15min | 10% | | |
| Phenylserinol | Time | | Phase B gradient | 210 | *Erythro*: 12.5 |
| | | 0min | 5% | | |
| | | 15min | 20% | | *Threo*: 13.4 |
| | | 18min | 30% | | |
| P-methyl phenylserinol | Time | | Phase B gradient | 220 | |
| | | 0min | 5% | | *Erythro*: 14.9 |
| | | 15min | 30% | | *Threo*: 15.6 |
| | | 22min | 30% | | |

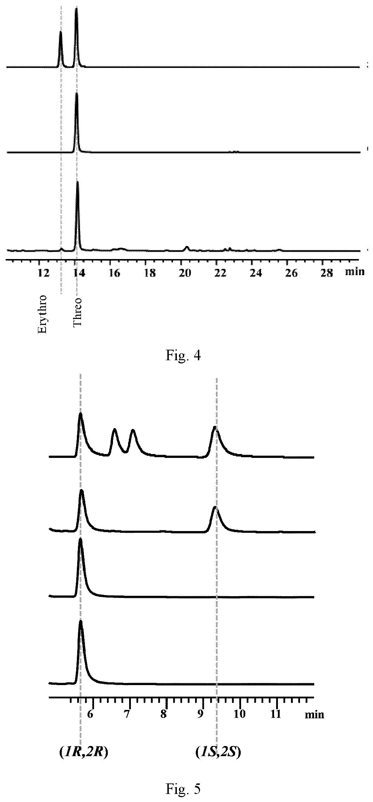

Fig. 4 shows the liquid phase of the reaction products by using p-methyl sulfonyl benzaldehyde as the substrate in Experimental Group 5 under C18 column conditions. In Fig. 4, the upper part shows the liquid phase of (erythro) - p-methyl sulfonyl phenylserinol and (threo) - standard p-methyl sulfonyl phenylserinol. The middle part shows the standard (1R, 2R) - p-methyl sulfonyl phenylserinol. The reaction products obtained in accordance with Experimental Group 5 are displayed at the bottom. As shown in Fig. 4, threo products are mainly prepared according to Experimental Group 5. According to the Formula: *de* = [(threo - erythro)/ (threo+erythro)] × 100%, the calculated result is *de* > 90%, where the erythro and threo represent (erythro) - and (threo) - p-methyl sulfonyl phenylserinol.

According to the liquid phase peaks, collect the (threo) - p-methylsulfonylphenylserinol product, concentrate it, and then separate it with a chiral liquid phase column to examine the enantioselectivity of the product. Analytical conditions for enantiomers: Chiral column IG, column temperature 25°C, 0.5ml/min, 224nm; Chromatographic conditions: Pure methanol (containing 0.1% diethylamine), 15 min. Fig. 5 shows the liquid phase of the (threo) - p-methyl sulfonyl phenylserinol product under chiral liquid phase column conditions. In Fig. 5, the top shows the control among standard erythro + threo products. The second line shows the control among standard threo products. The third line shows the control among standard (1R, 2R) - p-methyl sulfonyl phenylserinol products. The fourth and last line display the (threo) - p-methyl sulfonyl phenylserinol product. According to Fig. 5, the retention times for p-methyl sulfonyl phenylserinol (1R, 2R) and (1S, 2S) are 5.6 and 9.4 min, respectively. According to the Formula *ee* = [(RR-SS)/ (RR+SS)] × 100%, the calculated *ee* value is higher than 99%, where RR and SS represent (1R, 2R) - and (1S, 2S) - p-methyl sulfonyl phenylserinol.

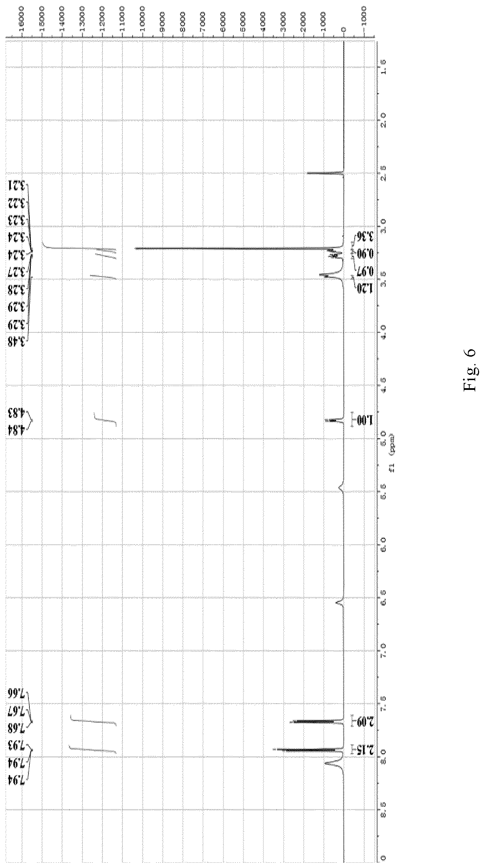

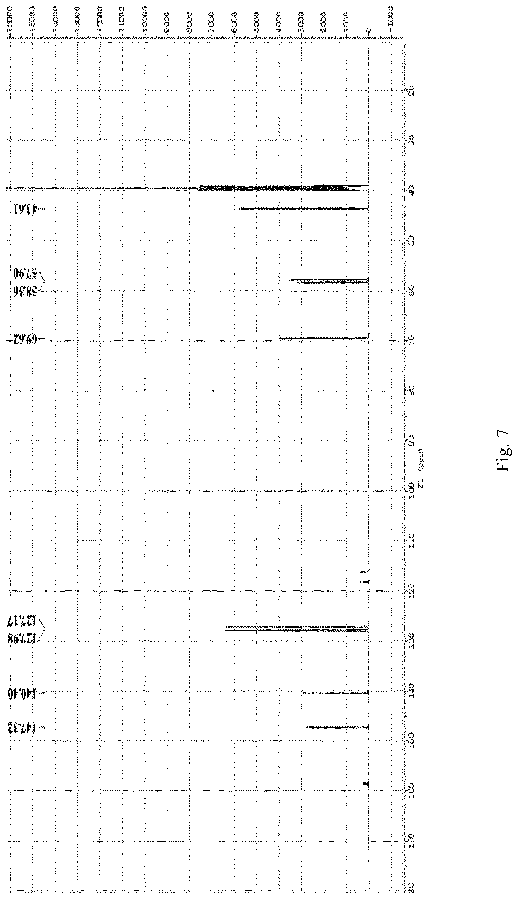

Fig. 6 shows the H-NMR of the reaction product (1R, 2R)-AMPP, and Fig. 7 shows the C-NMR of the reaction product (1R, 2R)-AMPP. According to the standard product control shown in Fig. 6-Fig. 7 and Fig. 4- Fig. 5, the (1R, 2R) - AMPP is synthesized based on the present invention. The (1R, 2R)-AMPP generated using the method described by the present invention has a high degree of stereoselectivity when one takes into account the de and ee values.

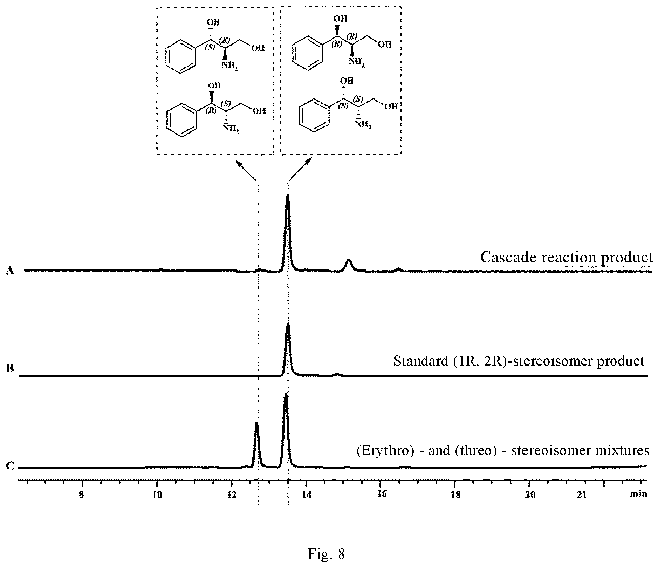

Fig. 8 shows the liquid phase of the reaction product obtained according to Experimental Group 9 by using benzaldehyde as the substrate under C18 column conditions. In Fig. 8, the upper part shows the reaction product obtained according to Experimental Group 9. The middle part shows the standard (1R, 2R) - methyl phenylserinol, and the lower part shows the standard (erythro) - phenylserinol and (threo) - phenylserinol products. Threo products are primarily made in accordance with Experimental Group 9 as shown in Fig. 5. According to the Formula: *de* = [(threo - erythro)/ (threo + erythro)] × 100%, the calculated result is *de* > 90%, where erythro and threo represent (erythro) - and (threo) - phenylserinol.

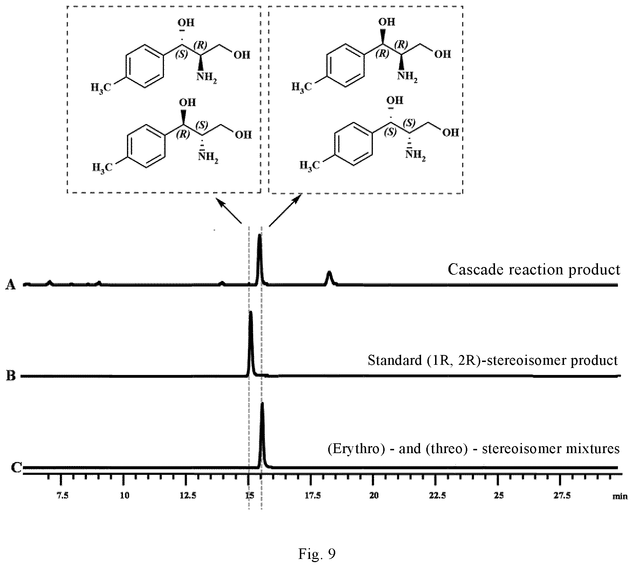

Fig. 9 shows the liquid phase of the reaction product obtained according to Experimental Group 13 by using p-methyl benzaldehyde as the substrate under C18 column conditions. In Fig. 9, the upper part shows the reaction product obtained according to Embodiment 13. The middle part shows the (erythro) -p-methyl phenylserinol, and the lower part shows the standard (threo) - p-methyl phenylserinol product. Threo products are primarily made in accordance with Experimental Group 13, as shown in Fig. 8. According to the Formula: *de* = [(threo - erythro)/(threo + erythro)] × 100%, the calculated result is *de >* 90%, where the erythro and threo represent (erythro) - and (threo) - p-methyl phenylserinol.

Amino acid sequence of Escherichia coli transketolase (SEQ ID NO: 11)

1 MSSRKELANA IRALSMDAVQ KAKSGHPGAP MGMADIAEVL WRDFLKHNPQ NPSWADRDRF
61 VLSNGHGSML IYSLLHLTGY DLPMEELKNF RQLHSKTPGH PEVGYTAGVE TTTGPLGQGI
121 ANAVGMAIAE KTLAAQFNRP GHDIVDHYTY AFMGDGCMME GISHEVCSLA GTLKLGKLIA
181 FYDDNGISID GHVEGWFTDD TAMRFEAYGW HVIRDIDGHD AASIKRAVEE ARAVTDKPSL
241 LMCKTIIGFG SPNKAGTHDS HGAPLGDAEI ALTREQLGWK YAPFEIPSEI YAQWDAKEAG

301 QAKESAWNEK FAAYAKAYPQ EAAEFTRRMK GEMPSDFDAK AKEFIAKLQA NPAKIASRKA
361 SQNAIEAFGP LLPEFLGGSA DLAPYNLTLW SGSKAINEDA AGNYIHYGVR EFGMTAIANG
421 ISLHGGFLPY TSTFLMFVEY ARNAVRMAAL MKQRQVMVYT HDSIGLGETG PTHQPVEQVA
481 SLRVTPNMST WRPCDQVESA VAWKYGVERQ DGPTALILSQ QNLAQQERTE EQLANIARGG
541 YVLKDCAGQP ELIFIATGSE VELAVAAYEK LTAEGVKARV VSMPSTDAFD KQDAAYRESV
601 LPKAVTARVA VEAGIADYWY KYVGLNGAIV GMTTFGESAP AELLFEEFGF TVDNVVAKAK
661 ELL

Nucleotide sequence of E. coli transketolase (SEQ ID NO: 12)

atgtcctcacgtaaagagcttgccaatgctattcgtgcgctgagcatggacgcagtacagaaagccaaatccggtcacccgggtgcccctatgggt
atggctgacattgccgaagtcctgtggcgtgatttcctgaaacacaacccgcagaatccgtcctgggctgaccgtgaccgcttcgtgctgtccaacg
gccacggctccatgctgatctacagcctgctgcacctcaccggttacgatctgccgatggaagaactgaaaaacttccgtcagctgcactctaaaact
ccgggtcacccggaagtgggttacaccgctggtgtggaaaccaccaccggtccgctgggtcagggtattgccaacgcagtcggtatggcgattgc
agaaaaaacgctggcggcgcagtttaaccgtccgggccacgacattgtcgaccactacacctacgccttcatgggcgacggctgcatgatggaag
gcatctcccacgaagtttgctctctggcgggtacgctgaagctgggtaaactgattgcattctacgatgacaacggtatttctatcgatggtcacgttga
aggctggttcaccgacgacaccgcaatgcgtttcgaagcttacggctggcacgttattcgcgacatcgacggtcatgacgcggcatctatcaaacg
cgcagtagaagaagcgcgcgcagtgactgacaaaccttccctgctgatgtgcaaaaccatcatcggtttcggttccccgaacaaagccggtaccca
cgactcccacggtgcgccgctgggcgacgctgaaattgccctgacccgcgaacaactgggctggaaatatgcgccgttcgaaatcccgtctgaaa
tctatgctcagtgggatgcgaaagaagcaggccaggcgaaagaatccgcatggaacgagaaattcgctgcttacgcgaaagcttatccgcaggaa
gccgctgaatttacccgccgtatgaaaggcgaaatgccgtctgacttcgacgctaaagcgaaagagttcatcgctaaactgcaggctaatccggcg
aaaatcgccagccgtaaagcgtctcagaatgctatcgaagcgttcggtccgctgttgccggaattcctcggcggttctgctgacctggcgccgtata
acctgaccctgtggtctggttctaaagcaatcaacgaagatgctgcgggtaactacatccactacggtgttcgcgagttcggtatgaccgcgattgct
aacggtatctccctgcacggtggcttcctgccgtacacctccaccttcctgatgttcgtggaatacgcacgtaacgccgtacgtatggctgcgctgat
gaaacagcgtcaggtgatggtttacacccacgactccatcggtctgggcgaaaccggcccgactcaccagccggttgagcaggtcgcttctctgcg
cgtaaccccgaacatgtctacatggcgtccgtgtgaccaggttgaatccgcggtcgcgtggaaatacggtgttgagcgtcaggacggcccgaccg
cactgatcctctcccaacagaacctggcgcagcaggaacgaactgaagagcaactggcaaacatcgcgcgcggtggttatgtgctgaaagactgc
gccggtcagccggaactgattttcatcgctaccggttcagaagttgaactggctgttgctgcctacgaaaaactgactgccgaaggcgtgaaagcgc
gcgtggtgtccatgccgtctaccgacgcatttgacaagcaggatgctgcttaccgtgaatccgtactgccgaaagcggttactgcacgcgttgctgta
gaagcgggtattgctgactactggtacaagtatgttggcctgaacggtgctatcgtcggtatgaccaccttcggtgaatctgctccggcagagctgct
gtttgaagagttcggcttcactgttgataacgttgttgcgaaagcaaaagaactgctgtaa

Amino acid sequence of the transaminase ATA117 (SEQ ID NO: 13)

1 MAFSADTSEI VYTHDTGLDY ITYSDYELDP ANPLAGGAAW IEGAFVPPSE ARISIFDQGY
61 LHSDVTYTVF HVWNGNAFRL DDHIERLFSN AESMRIIPPL TQDEVKEIAL ELVAKTELRE
121 AFVSVSITRG YSSTPGERDI TKHRPQVYMY AVPYQWIVPF DRIRDGVHAM VAQSVRRTPR
181 SSIDPQVKNF QWGDLIRAVQ ETHDRGFEAP LLLDGDGLLA EGSGFNVVVI KDGVVRSPGR
241 AALPGITRKT VLEIAESLGH EAILADITLA ELLDADEVLG CTTAGGVWPF VSVDGNPISD
301 GVPGPVTQSI IRRYWELNVE SSSLLTPVQY

Nucleotide sequence of the transaminase ATA117 (SEQ ID NO: 14)

atggcatttagcgcggataccagcgaaatcgtctatacccacgataccggtctggattacatcacctacagcgattacgaactggatccggcaaatccg
ttagcaggcggcgcagcttggattgaaggcgcatttgttccgccgtctgaagcgcgtattagcatcttcgatcagggctatctgcatagcgacgttacct
ataccgtcttccacgtttggaacggtaacgcatttcgtctggacgatcacatcgaacgtctgttcagcaacgccgaaagtatgcgtattattccgccgctg
acccaagacgaagtcaaagaaatcgcgctggaactggttgcaaaaaccgaactgcgcgaagcgtttgttagcgttagcattacccgcggttatagtag
taccccgggcgaacgcgatattaccaaacatcgtccgcaggtctacatgtacgctgttccgtaccagtggatcgttccgtttgatcgtattcgcgacggc
gttcacgcaatggttgcacagagcgtgcgtcgtaccccgcgtagcagcatcgatccgcaggtcaaaaacttccagtggggcgatttaattcgcgcagt
tcaggaaacccacgatcgcggttttgaagcaccgttactgttagacggcgacggtttactggcagaaggtagcggctttaacgtcgtcgtcattaaaga
tggggttgttcgttctccgggtcgtgcagcattaccgggtattacccgcaaaaccgttctggaaattgcggaatccctgggtcatgaagcgattctggcg
gatattaccttagcggaactgctggacgcagacgaagttttaggttgtaccaccgctggcggcgtttggccgtttgttagcgttgacggcaatccgatta

gcgatggtgttccgggtccggttacccaaagcattattcgtcgctactgggagctgaacgttgaaagtagcagcctgttaaccccggttcagtat

The present invention has provided the disclosure in preferred Embodiments. Nevertheless, they are not meant to hinder the current invention. Anyone skilled in this technology can make changes and modifications within the present's invention spirit and scope. Therefore, the claims' defined scope should be the foundation for protecting the current invention.

## Claims

1. An enantioselective flipped ω-transaminase ATA117 mutant, which is **characterized in that** the protein amino acid sequence of the said ω-transaminase ATA117 mutant is represented by SEQ ID NO: 1.

2. The ω-transaminase ATA117 mutant, as defined in Claim 1, which is **characterized in that** the said ω-transaminase ATA117 mutant generated by the amino acid sequence of the ω-transaminase ATA117 through iterative saturation mutation; The said ω-transaminase ATA117 mutant has at least a mutation of the valine, phenylalanine, isoleucine, or phenylalanine at Sites N1, N2, N3, or N4, respectively.

3. The ω-transaminase ATA117 mutant, as defined in Claim 2, which is **characterized in that** the valine at Site N1 is mutated to alanine, the phenylalanine at Site N2 is mutated to cysteine, the isoleucine at Site N3 is mutated to histidine, and the phenylalanine at Site N4 is mutated to histidine.

4. A kind of DNA nucleotide sequence, which is **characterized in that** the said DNA nucleotide sequence contains a nucleotide sequence for coding a ω-transaminase mutant, as described in Claim 2.

5. A kind of DNA nucleotide sequence, which is **characterized in that** the said DNA nucleotide sequence represented by SEQ ID NO: 2, as described in Claim 4.

6. A kind of plasmid with the DNA nucleotide sequence as defined in Claim 5.

7. A genetically engineered bacterium expressing the ω-transaminase ATA117 mutant, which is **characterized in that** the protein amino acid sequence of the ω-transaminase ATA117 mutant is represented by SEQ ID NO: 1.

8. A method for constructing a genetically engineered bacterium expressing the ω-transaminase ATA117 mutant as defined in Claim 7, which is **characterized by** the following steps: A1, Taking the carried recombinant plasmid for coding the gene of ATA117 as the template; A2, designing and synthesizing the primer containing the mutation; A3, performing reverse PCR amplification of the whole-plasmid; and A4, transforming the PCR product to express the host.

9. A method for constructing a genetically engineered bacterium expressing the ω-transaminase ATA117 mutant, defined in Claim 8, which is **characterized in that** the expression host *E.coli* BL21 (DE3).

10. A method for producing (1R, 2R) -1,3-dihydroxy-2-amido-1-p-methyl sulfonyl phenyl propane 2b based on the ω-transaminase ATA117 mutant as defined in Claim 1: With the pure protein of the ω-transaminase ATA117 mutant as the catalyst, the racemization of 1,3-dihydroxy-1-p-methyl sulfonyl propiophenone 1 can be catalyzed in a buffer system; In addition, the high stereoselective (1R, 2R) -1, 3-dihydroxy-2-amido-1-p-methyl sulfonyl phenylpropane 2b can be obtained through kinetic resolution.

11. An transaminase mutant, is **characterized by** the transaminase mutant has amino acid mutation sequence as represented by SEQ ID NO: 13, and the amino acid mutation Site is one of the following combinatorial mutation Sites: V69F, V69G, V69A, V69L, V69I, V69P, V69M, V69W, V69S, V69Q, V69T, V69C, V69N, V69Y, V69D, V69E, V69K, V69R, V69H, V69A+F122G, V69A+F122A, V69A+F122L, V69A+F122I, V69A+F122V, V69A+F122P, V69A+F122M, V69A+F122W, V69A+F122S, V69A+F122Q, V69A+F122T, V69A+F122C, V69A+F122N, V69A+F122Y, V69A+F122D, V69A+F122E, V69A+F122K, V69A+F122R, V69A+F122H, V69A+F122C+I157F, V69A+F122C+I157G, V69A+F122C+I157A, V69A+F122C+I157L, V69A+F122C+I157I, V69A+F122C+I157V, V69A+F122C+I157P, V69A+F122C+I157M, V69A+F122C+I157W, V69A+F122C+I157S, V69A+F122C+I157Q, V69A+F122C+I157T, V69A+F122C+I157C, V69A+F122C+I157N, V69A+F122C+I157Y, V69A+F122C+I157D, V69A+F122C+I157E, V69A+F122C+I157K, V69A+F122C+I157R, V69A+F 122C+115 7H, V69A+F122C+I157H+F225G, V69A+F122C+I157H+F225A, V69A+F122C+I157H+F225L, V69A+F122C+I157H+F225I, V69A+F122C+1157H+F225V, V69A+F122C+I157H+F225P, V69A+F122C+I15 7H+F225M, V69A+F122C+I15 7H+F225W, V69A+F122C+I157H+F225S, V69A+F122C+1157H+F225Q, V69A+F122C+I157H+F225T, V69A+F122C+I157H+F225C, V69A+F122C+I15 7H+F225N, V69A+F122C+1157H+F225Y, V69A+F122C+I157H+F225D, V69A+F122C+I157H+F225E, V69A+F122C+1157H+F225K, V69A+F122C+I157H+F225R, V69A+F122C+1157H+F225H.
